**Europäisches Patentamt**

**European Patent Office**

**Office européen des brevets**

(11) Veröffentlichungsnummer: **0 154 282**
**A2**

# EUROPÄISCHE PATENTANMELDUNG

(21) Anmeldenummer: 85102078.4

(22) Anmeldetag: 26.02.85

(51) Int. Cl.⁴: **A 61 B 10/00**

(30) Priorität: 01.03.84 DE 3407646

(43) Veröffentlichungstag der Anmeldung:
11.09.85 Patentblatt 85/37

(84) Benannte Vertragsstaaten:
BE DE FR GB IT LU NL

(71) Anmelder: Pfeiffer, Gottfried Prof.-Dr.
Hufeisenstrasse 13
D-8057 Eching(DE)

(72) Erfinder: Pfeiffer, Gottfried Prof.-Dr.
Hufeisenstrasse 13
D-8057 Eching(DE)

(74) Vertreter: Hoeger, Stellrecht & Partner
Uhlandstrasse 14c
D-7000 Stuttgart 1(DE)

(54) Vorrichtung zur Entnahme von Materialproben.

(57) Um bei einer Vorrichtung zur Entnahme von Materialproben, insbesondere zur Entnahme von tierischen Muskel-oder Gewebeproben, mit einem Rundschneid-zylinder (2), der an seinem einen Ende eine angeschliffene Schneide (1) aufweist, die Entnahme möglichst gewichtsgleicher und/oder formgleicher Gewebeproben ohne Verformung und Zerstörung der Struktur des Materials zu erlauben, wird vorgeschlagen, daß der Rundschneidzylinder (2) um seine Längsachse rotierbar an einer Haltevorrichtung (8) befestigt ist und daß eine den Rundschneidzylinder (2) in Drehbewe-gung versetzende Drehvorrichtung (6) vorgesehen ist.

EP 0 154 282 A2

./...

Croydon Printing Company Ltd.

FIG. 1

1

Professor Dr. G. Pfeiffer, 8057 Eching

Vorrichtung zur Entnahme von

Materialproben

Die Erfindung bezieht sich auf eine Vorrichtung zur Entnahme von Materialproben, insbesondere zur Entnahme von
tierischen Muskel- oder Gewebeproben nach dem Oberbegriff des Anspruches 1.

Vor allem auf dem Gebiet der Materialprüfung und der Probenentnahme für Analysen kommt es häufig darauf an, Probenmaterial möglichst formgleich, gewichtsgleich oder
form- und gewichtsgleich zu entnehmen. Dies ist bei zähelastischen Materialien, wie einer Gummimatte, oder bei
Material mit spröder Struktur, wie bei Kochschokolade,
oder bei Produkten von komplexer grobgeweblicher Zusammensetzung, wie Fleisch, oder bei Produkten heterogener
Struktur und Textur sowie Konsistenz, wie bei Backwaren,

-2-

oder bei einem in mehreren, miteinander verbundenen Schichten ausgefrorenen Probengut, wie einem vor dem Frosten längere Zeit sedimentierten Klärschlamm, problematisch und mit herkömmlichen Stanzen ohne Verformungen oder Zerstörungen der Strukturen nicht möglich.

Bislang wurden derartige Materialproben mit Hilfe einer einfachen Zylinderstanze, welche an ihrem einen Ende eine angeschliffene Schneide besitzt, durchgeführt. Mit dieser Zylinderstanze ist es jedoch nicht möglich, einem Material, wie beispielsweise einem Stück Fleisch mit einem gewissen Gehalt an Fettgewebe und straffem Bindegewebe, eine einem Soll-Gewicht möglichst nahekommende Probe zu entnehmen. Die Probenentnahme mit einer Zylinderstanze läßt sich nur durch Aufwendung eines großen Stanzdruckes oder -schlages und daher nicht ohne unerwünschte Verformung sowie Zerstörung von Struktur und Textur und meist auch nicht ohne Benachteiligung der Gewichtskonstanz erreichen. Auch ist anschließend die Entnahme der Materialprobe aus der Zylinderstanze selbst relativ schwierig, da die Probe im Inneren des Stanzenzylinders leicht festklemmt oder festklebt.

Aufgabe der Erfindung ist es, eine Vorrichtung der genannten Gattung bereitzustellen, welche die Entnahme möglichst gewichtsgleicher oder möglichst formgleicher oder möglichst gewichts- und formgleicher Materialteile ohne Verformung oder Zerstörung der Struktur erlaubt. Des weiteren soll die Vorrichtung einen einfachen Aufbau aufweisen und eine leichte und sichere Handhabung ermöglichen.

Diese Aufgabe wird durch eine Vorrichtung mit den Merkmalen des Anspruches 1 gelöst.

Demgemäß weist die erfindungsgemäße Vorrichtung einen
Rundschneidezylinder auf, der an seinem einen Ende eine
angeschliffene Schneide besitzt, und ist dadurch gekennzeichnet, daß der Rundschneidezylinder um seine Längsachse rotierbar an einer Haltevorrichtung befestigt ist
und daß eine den Rundschneidezylinder in Drehbewegung
versetzende Drehvorrichtung vorgesehen ist.

Hierdurch wird erreicht, daß der Rundschneidezylinder,
also das Schneidewerkzeug während des Ausschneidens der
Materialprobe in eine Drehbewegung versetzt wird, wodurch eine echte, saubere Schneidwirkung erzielt wird,
ohne daß ein besonderer, die Struktur beschädigender
Druck auf das Material ausgeübt werden muß.

Gemäß einer Weiterbildung des Erfindungsgedankens ist
von Vorteil, daß am rückwärtigen Ende des Rundschneidezylinders zwecks Aufnahme der ausgeschnittenen Materialproben ein Sammelbehälter lösbar befestigt ist. Die Befestigung des Sammelbehälters kann in an sich bekannter
Weise über einen Bajonett- oder Schraubverschluß vorgenommen werden. Diese Verschlußarten weisen gleichzeitig
den Vorteil auf, daß ein ebenfalls mit Bajonettverschluß
oder Schraubgewinde versehener Deckel zum Verschließen
des abgenommenen Behälters verwendet werden kann. Der
Sammelbehälter kann dabei, wenn er für die Entnahme von
Skelettmuskelproben für die Untersuchung auf Trichinen
bestimmt ist, so ausgelegt sein, daß er zumindest 25 g

- 4 -

Probengut aufzunehmen vermag und nach Verschluß mit einem
Deckel mit Bajonettverschluß oder Schraubgewinde zur Weitergabe des Probenmaterials an ein Laboratorium, beispielsweise mit Rohrpost, geeignet ist.

Es ist von Vorteil, wenn der Rundschneidezylinder, in
axialer Richtung gesehen, zweigeteilt ist. Dabei ist der
erste, vordere Teil ein Reduzierzylinder, welcher gleichzeitig der Schneidzylinder ist und je nach Material und
Größe der zu entnehmenden Probe eine unterschiedliche
lichte Weite oder auch eine unterschiedliche Schneide
aufweisen kann. Der zweite, rückwärtige Teil ist ein
Drehzylinder, welcher in Verbindung steht mit der Drehvorrichtung und dem Sammelbehälter und gleichzeitig zur
Befestigung an der Haltevorrichtung dient. Drehzylinder
und Reduzierzylinder sind ebenfalls über Bajonett- oder
Schraubverschlüsse leicht lösbar miteinander verbunden.
So ist die Möglichkeit gegeben, daß der Reduzierzylinder
je nach Bedarf schnell und leicht ausgetauscht werden
kann. Wie bereits erwähnt, kann die Schneide des Rundschneidezylinders je nach zu schneidendem Material unterschiedlich ausgebildet sein, so daß eine glatte Schneide oder eine Sägezahnschneide vorhanden sein kann.

Von besonderem Vorteil ist, wenn die erfindungsgemäße
Vorrichtung eine Haltevorrichtung aufweist, welche deren Handhabung wesentlich erleichtert.

Die Haltevorrichtung kann in erster Ausführung ein senkrecht zum Rundschneidezylinder verlaufender Griffarm sein.

In einer weiteren Ausführung kann die Einheit aus Rundschneidezylinder und Sammelbehälter auch an ein Tischstativ montiert sein. Sie kann aber auch freibleibend
von einer Verbindung an eine Hilfsvorrichtung benutzt
werden. Der Amboß ist in diesem Fall vorteilhaft in eine Tischplatte eingelassen.

Die Haltevorrichtung kann in ihrer vorteilhaftesten Ausführungsform die Form einer Zange aufweisen, an deren
erstem Schenkelteil der Rundschneidezylinder mit Sammelbehälter drehbar befestigt ist und an deren zweitem
Schenkelteil ein mit der Schneide des Rundschneidezylinders zusammenwirkendes Gegendruckstück angeordnet
ist. Durch die zangenförmige Haltevorrichtung ist eine
leichte Handhabung der Einrichtung sichergestellt. Es
besteht die Möglichkeit, mit einer Hand in einfacher
Weise einem Material eine Probe zu entnehmen.

Das Gegendruckstück kann dabei ein Amboß sein, welcher
mindestens eine mit der Schneide des Rundschneidezylinders zusammenwirkende Ringvertiefung aufweist. Der Amboß kann jedoch auch mehrere konzentrisch zueinander
liegende Ringvertiefungen aufweisen, welche den Einsatz
von Reduzierzylindern unterschiedlichen Durchmessers erlauben, ohne daß der Amboß ebenfalls ausgetauscht werden müßte. Der Schneiderand des jeweiligen Rundschneidezylinders fügt sich in die Ringvertiefungen ein, wenn
die Zange geschlossen wird. Das Profil der Ringvertiefungen ist vorteilhafterweise so gestaltet, daß die
Schneide des jeweils benutzten Rundschneidezylinders
automatisch nachgeschliffen wird, wenn der Schneiderand des Schneidezylinders in die Ringvertiefung eingreift.

Der Amboß kann dabei aus einem härteren Metall bestehen
als der Rundschneidezylinder oder der Reduzier-Rundschneidezylinder. Er kann jedoch auch aus einem leicht auswechselbaren, relativ weichen Kunststoffmaterial bestehen.
In diesem letzteren Fall entstehen die das Durchschneiden z.B. faseriger Strukturgebilde von höherer mechanischer Festigkeit, wie z.B. Leder, begünstigenden Ringvertiefungen von selbst. Sie machen den Amboß im Laufe einer längeren Benutzung unbrauchbar. Hierfür ist der
Kunststoffamboß auswechselbar an der Haltevorrichtung
befestigt.

Um die Repräsentanz und Gewichtskonstanz des zu entnehmenden Probengutes zu erhöhen, ist es von Vorteil, am
Amboß eine Hilfsvorrichtung vorzusehen. Der Amboß kann
in diesem Fall das Ende des kurzen Schenkelstückes selbst
sein. Bei Prüfgütern nämlich, die nicht zwei annähernd
parallele Oberflächen, sondern einen keilförmigen, konischen oder unregelmäßigen Umriß haben, oder bei Prüfgütern, wie beispielsweise weichen Organgeweben von Tieren oder Pflanzen, die schon durch geringste Kräfte verformt werden, kann eine höhere Repräsentanz und Gewichtskonstanz des zu entnehmenden Probenmaterials dadurch erzielt werden, daß beidseitig und planparallel am Amboß
höhenverstellbare Wangen angebracht werden, mit denen
sich das Gewicht des ausgeschnittenen Probenmaterials in
einem engen Streubereich festlegen läßt. So läßt sich
z.B. ein konisch zulaufendes Muskelstück so zwischen die
Wangen fügen, daß es sich diesen und dem Amboß dicht anschmiegt und dabei den oberen Rand der Wangen eben erreicht, jedoch nicht überschreitet.

Die Vorrichtung dient insbesondere der Entnahme von Skelettmuskelproben aus der sogenannten Zwerchfellpfeiler-Muskulatur geschlachteter Schweine für die Untersuchung auf das Vorhandensein von Trichinen. Am ausgewaideten, hängenden Schwein läßt sich die Zwerchfellpfeiler-Muskulatur für Probenentnahme in der Weise vorbereiten, daß beim Auswaiden des Tierkörpers das Zwerchfell im Bereich der beiden Zwerchfellpfeiler als ein etwa 5 cm langer und 10 cm breiter Muskelsaum dem Tierkörper anbelassen wird. In diesem Muskelsaum werden die beiden konisch verdickten Zwerchfellpfeiler voneinander und von der übrigen Zwerchfellmuskulatur mit Längsschnitten isoliert, so daß sie nun als verdickte Stümpfe nach unten hängen.

Zur serienmäßigen Probenentnahme am Schlachtband wird vorzugsweise die mit einem Amboß mit höhenverstellbaren Seitenwangen ausgestattete zangenförmige Vorrichtung benutzt. Die Benutzung geschieht in der Weise, daß die entnehmende Person mit der einen Hand zunächst den einen der beiden isolierten Zwerchfellpfeiler zwischen die Seitenwangen des Ambosses einpaßt und mit der anderen Hand schließlich an derjenigen Stelle des Muskelbauchstumpfes die Probe durch Schließen der Zange ausschneidet, an der das der Höhenstellung der Seitenwangen entsprechende Probe-Minimalgewicht von beispielsweise 0,6 g gewährleistet ist. Anschließend wird dieser Entnahmevorgang am anderen Zwerchfellpfeiler auf dieselbe Weise
wiederholt. In statistischen Reihenversuchen wurde so
erhärtet, daß das auf diese Weise je Tier entnommene
Probenmaterial mit an Sicherheit grenzender Wahrscheinlichkeit ein Minimalgewicht 1,17 g und ein Maximalgewicht von 1,7 g aufweist, wenn ein Rundschneidezylinder mit einem Durchmesser von 10 mm verwendet wird.
Routinierten Probennehmern ist es möglich, die Streuung der Einzelgewichte der so entnommenen Proben auch
ohne Benutzung eines Amboß mit höhenverstellbaren Seitenwangen so gering zu halten, daß das Minimalgewicht
von 1,2 g und das Maximalgewicht von 2,1 g gewährleistet ist.

Erfindungsgemäß kann die Vorrichtung eine Auswurfeinrichtung aufweisen. Diese Auswurfeinrichtung dient zum
Entfernen der geschnittenen Materialproben aus der
lichten Weite des Rundschneidezylinders und zum Einbringen dieser Probe in den Sammelbehälter, ohne daß die Bedienungsperson noch zusätzlich Vorkehrungen treffen muß.

Die Auswurfeinrichtung kann im Amboß vorgesehen sein,
und dabei aus einer im Amboß angeordneten zentrischen
Öffnung bestehen, welche über ein Ventil mit einer Druckleitung in Verbindung steht. Gleichzeitig weist der Sammelbehälter Öffnungen auf, die als Druckausgleich für die
eingebrachte Druckluft dienen. Das Überdruckventil kann
sich vorteilhafterweise bei Aufwendung eines beim Schließen der Zange bestimmten mechanischen Drucks plötzlich
öffnen und einem Druckluftstoß den Weg in den Rundschneidezylinder freigeben. Durch den Druckluftstoß wird das im Rundschneidezylinder befindliche Material in den Sammelbehälter transportiert. Das Öffnen des Druckventils kann aber auch elektro-mechanisch dadurch erfolgen, daß der Rundschneidezylinder den Amboß lediglich berührt und hierdurch einen Stromkreis schließt. Die Auswurfeinrichtung kann in anderer Ausführungsweise aus einem im Sammelbehälter angeordneten, gelochten Rohr bestehen, welches mit einem Unterdruckrohr in Verbindung steht. Eine zentrische Öffnung im Amboß kann dabei als Druckausgleichsöffnung dienen. Der Sammelbehälter ist in diesem Fall ohne Öffnungen und vakuumstabil, so daß sich ein entsprechendes Vakuum, welches die ausgeschnittenen Proben in das Innere des Sammelbehälters befördert, aufbauen kann.

Als Auswurfseinrichtung kann auch im Amboß axial beweglicher Stempel vorgesehen sein, welcher über einem elastischen Stahlkabel mit einem am Zangenschenkel verschieblich angeordneten Schiebeknopf verbunden ist. Durch Verschieben des Schiebeknopfes wird der Stempel aus der Stirnfläche des Amboß herausgedrückt und gleitet dabei in den Rundschneidezylinder, wodurch das im Rundschneidezylinder befindliche Material in den Sammelbehälter gestoßen wird.

Hier sei noch darauf hingewiesen, daß zangenförmige Vorrichtungen zum Perforieren, also zum Einbringen von kreisrunden Öffnungen, beispielsweise in Leder, bereits bekannt sind. Auch bei diesen bekannten Zangen-Lochern ist am einen Schenkel ein Schneidezylinder angebracht, welcher an seinem vorderen Ende eine Schneide aufweist,
während am zweiten Schenkel ein mit dem Schneidezylinder
zusammenwirkendes Gegendruckstück (Amboß) angeordnet
ist. Mit Hilfe dieser zangenförmigen Schneideeinrichtung
werden die kreisrunden Öffnungen in das zwischengeschobene Material durch reine vertikale Preßbewegung eingespannt. Es ist folglich der eingangs beschriebene Nachteil gegeben, daß unerwünschte Verformungen sowie eine
Zerstörung der Struktur und Textur erhalten wird. Des
weiteren können die ausgestanzten Butzen nicht ohne weiteres dem Zylinder der Schneideinrichtung entnommen werden. Die ausgestanzten Butzen werden immer jeweils vom
nachfolgend ausgestanzten Teil im Schneidezylinder nach
rückwärts geschoben, bis sie am rückwärtigen Ende des
Zylinders herausfallen. Insbesondere für zähe und spröde und strukturkomplexe Materialien vom Typ einer an
kollogenem oder elastischem Bindegewebe reichen Muskulatur ist diese bekannte Lochstanz-Vorrichtung nicht geeignet. Auch können die ausgeschnittenen Proben nicht in
relativ sauberer und hygienischer Weise eingesammelt und
anschließend eingeschlossen und transportiert werden.

Gemäß einer Ausführungsform der erfindungsgemäßen Vorrichtung kann die Drehvorrichtung elektrisch oder elektromagnetisch betrieben werden. Hierfür ist eine Ankerwicklung um den Rundschneidezylinder gelegt, der in
diesem Fall gleichzeitig die Ankerwelle darstellt.

Die Drehvorrichtung kann auch eine pneumatische Einrichtung sein, wobei Turbinenschaufeln zwecks Erzeugung der Drehbewegung an dem Rundschneidezylinder bzw. Drehzylinder angeordnet sind.

Die Drehvorrichtung kann jedoch auch eine rein mechanische Einrichtung sein. Hierfür besitzt sie in ihrer einfachsten Ausführungsform einen Rundschneidezylinder, der
von Hand gedreht wird. Dies ist auf verschiedenste Weise
durchführbar. So ist dies z.B. dadurch möglich, daß der
Rundschneidezylinder so an den Sammelbehälter gekoppelt
ist, daß eine Drehung des Sammelbehälters mit der freien
Hand den Rundschneidezylinder mitdreht. Es kann jedoch
auch am Rundschneidezylinder ein Drehring oder es können Hebel befestigt  sein, welche es erlauben, den Rundschneidezylinder in eine Drehbewegung zu versetzen.

Am Rundschneidezylinder kann auf der Mantelfläche auch
ein Zahnkranz befestigt sein, welcher mit einer in dem
den  Rundschneidezylinder tragenden Schenkel der Haltevorrichtung längsverschieblichen Zahnstange zusammenwirkt. Diese Zahnstange greift über eine Verzahnung an
ihrem einen Ende in das Zahnritzel des Rundschneidezylinders ein, während das andere, aus dem Schenkel herausragende Ende mit einer Fingeröse versehen ist, über
welche die Zahnstange hin und her geschoben und so der
Rundschneidezylinder in eine pendelnde Schneidebewegung
versetzt wird. All diese aufgezählten mechanischen Drehvorrichtungen benötigen jedoch eine Zwei-Hand-Bedienung.
Die Bedienungsperson muß folglich mit der einen Hand die
Schneideeinrichtung ansetzen und halten und mit der zweiten Hand die Drehvorrichtung betätigen.

Zwecks Ein-Hand-Betätigung kann die mechanische Drehvorrichtung ebenfalls einen Zahnkranz am Rundschneidezylinder aufweisen. In dem den Rundschneidezylinder tragenden Zangenschenkel ist eine Welle drehbar befestigt, welche an einer Seite ein Ritzel trägt, welches mit dem Zahnkranz des Rundschneidezylinders getrieblich verbunden ist, während am anderen Ende ein Ritzel vorgesehen ist, welches mit dem einen Ende einer Zahnstange zusammenwirkt, deren anderes Ende am äußeren Ende des zweiten Zangenschenkels befestigt ist. Durch Zusammendrücken der zangenförmigen Haltevorrichtung, also bei Anliegen der Vorrichtung an dem Material, aus welchem eine Probe entnommen werden soll, werden die beiden Schenkel der Haltevorrichtung einander genähert, wodurch die Zahnstange auf dem unteren Ritzel des zweiten Schenkels entlanggleitet. Hierdurch wird die Welle und das vordere Ritzel mitgedreht und somit über den Zahnkranz der Rundschneidezylinder in eine Drehbewegung versetzt. Durch einfaches Zusammendrücken der Haltevorrichtung mit einer Hand vermag die Bedienungsperson folglich eine optimale Probenentnahme durchzuführen. Von besonderem Vorteil kann dabei sein, wenn das äußere Ende des Zangenschenkels, an welchem die Zahnstange befestigt ist, über ein Gelenk mit dem restlichen Teil des Schenkels verbunden ist. Eine Blattfeder sorgt dabei für die gerade Ausrichtung der beiden Schenkelteile zueinander, wenn die Vorrichtung nicht betätigt oder wenn der Schneidezylinder nicht in Drehbewegung versetzt werden soll. Beim Schließen der Zange gleitet die Zahnstange über das untere Ritzel der Welle und setzt diese bei Aufwendung einer etwas größeren Kraft infolge einer Abbeugung des Gelenks auch dann noch in Bewegung, wenn die Schneide des Rundschneidezylinders bereits die Ringvertiefung des Amboß erreicht hat. Des weiteren kann über das Schwenkteil des Zangenschenkels der Rundschneidezylinder in eine schnellere Drehbewegung versetzt werden, als bei starrer Ausführung des Schenkels. Die Zahnstange kann jedoch auch über das Schwenkteil des Schenkels in eine Hin- und Herbewegung versetzt werden, welche in eine Hin- und Her-Drehbewegung des Rundschneidezylinders umgewandelt wird, je nach dem, was für ein Material geschnitten oder wie schnell die Probenentnahme stattfinden soll.

Bei einem weiteren bevorzugten Ausführungsbeispiel ist vorgesehen, daß der Rundschneidzylinder axial verschieblich gelagert und durch Federmittel bis zu einem Anschlag in Richtung auf das Gegendruckstück verschoben ist und daß eine Zwangsführung vorgesehen ist, die den Rundschneidzylinder beim Verschieben gegen die Kraft der Federmittel um seine Längsachse in Drehung versetzt.

Diese Zwangsführung kann beispielsweise eine wendelförmige Führung am Rundschneidezylinder oder an dessen Lagerung und einen in diese Führung eingreifenden Vorsprung am jeweils anderen Teil aufweisen.

Es ist auch möglich, daß Rundschneidzylinder und Lagerung zur Zwangsführung ineinandergreifende, wendelförmige Rippen aufweisen.

Durch eine solche Ausgestaltung wird die Drehbewegung des Rundschneidzylinders zwangsläufig erzeugt, wenn der Rundschneidzylinder gegen das Gegendruckstück gedrückt wird, da das Gegendruckstück über das dazwischenliegende zu schneidende Gut den Rundschneidzylinder gegen die Kraft der Federmittel in die Lagerung einschiebt. Durch die Zwangsführung führt diese axiale Verschiebung des Rundschneidzylinders in der Lagerung zu einer Drehung des Rundschneidzylinders um seine Längsachse, ohne daß dazu zusätzliche, komplizierte Antriebs- oder Getriebemittel notwendig sind.

Nachfolgend wird die Erfindung anhand von Ausführungsbeispielen unter Bezug auf die Zeichnung näher beschrieben.

Es zeigt:

Figur 1    eine Draufsicht auf eine erfindungsgemäße Vorrichtung in erster Ausführung,

Figur 2    einen Schnitt nach den Linien II-II aus Figur 1,

Figur 3    einen teilweisen Schnitt durch einen Rundschneidezylinder in zweigeteilter Ausführungsform,

Figur 4    eine Perspektivansicht auf eine Hilfsvorrichtung am Amboß der erfindungsgemäßen Vorrichtung,

Figur 5    eine erfindungsgemäße Vorrichtung mit einer Drehvorrichtung in rein mechanischer
           Ausführungsweise,

Figur 6    eine teilweise Ansicht auf eine erfin-
           dungsgemäße Vorrichtung, die eine mecha-
           nische Dreheinrichtung in anderer, ein-
           facherer Ausführungsweise umfaßt und

Figur 7    einen Längsschnitt durch den Rundschneid-
           zylinder und dessen Lagerung bei einem
           weiteren bevorzugten Ausführungsbeispiel.

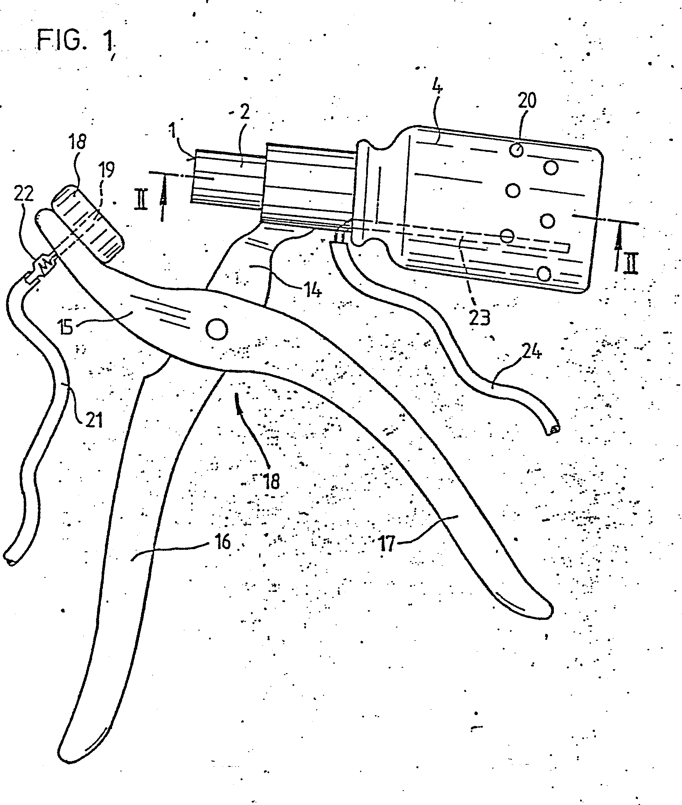

Die in Figur 1 dargestellte erste Ausführungsform der erfindungsgemäßen Vorrichtung weist eine zangenförmige Haltevorrichtung 8 auf, welche zwei lange Schenkel 16 und 17
zum Greifen und Betätigen der Vorrichtung besitzt. Die
kurzen Schenkelteilstücke 14 und 15 tragen an ihren äußeren Enden jeweils Vorrichtungsteile, die z.T. miteinanderwirkend angeordnet sind. So ist am kurzen Teilstück 14
ein Rundschneidezylinder 2 derart befestigt, daß er mit
seinem Schneiderand 1 auf das gegenüberliegende Schenkelteilstück 15 zeigt. Am rückwärtigen, in der Zeichnung
rechtsliegenden Ende des Rundschneidezylinders 2 ist ein
Sammelbehälter 4 zur Aufnahme der entnommenen Proben lösbar angebracht. Im Sammelbehälter 4 ist ein Rohr 23 angeordnet, welches Perforierungen aufweisen kann und mit einem Unterdruck-Schlauch (24) in Verbindung steht. In der
Mantelfläche des Sammelbehälters 4 können Öffnungen 20
als Druckausgleichsöffnungen im Falle der Verwendung ei-

-16-

ner mit Druckluft vom Amboß aus arbeitenden Auswerfeinrichtung angeordnet sein. Am anderen Schenkelteilstück
15 ist ein Amboß 18 befestigt, an dessen dem Rundschneidzylinder zuweisenden Seite Ringvertiefungen, in welche
der Schneiderand 1 eingreifen kann, eingebracht sind. Im
Amboß ist zentrisch eine axiale Öffnung vorgesehen, welche über ein an der dem Rundschneidezylinder entgegengesetzten Seite angeordnetes Überdruckventil 22 mit einer
Druckluftleitung 21 in Verbindung steht. Die Öffnung 19
ist auch dann im Amboß vorgesehen, wenn die Druckluftleitung 21 nicht vorhanden ist, also nicht mit Druckluft
die Proben ausgeworfen werden. Sie dient dann als Druckausgleichsöffnung, wenn die Proben mit Hilfe von Unterdruck (Rohr 23, Schlauch 24) in den Sammelbehälter 4 eingebracht werden.

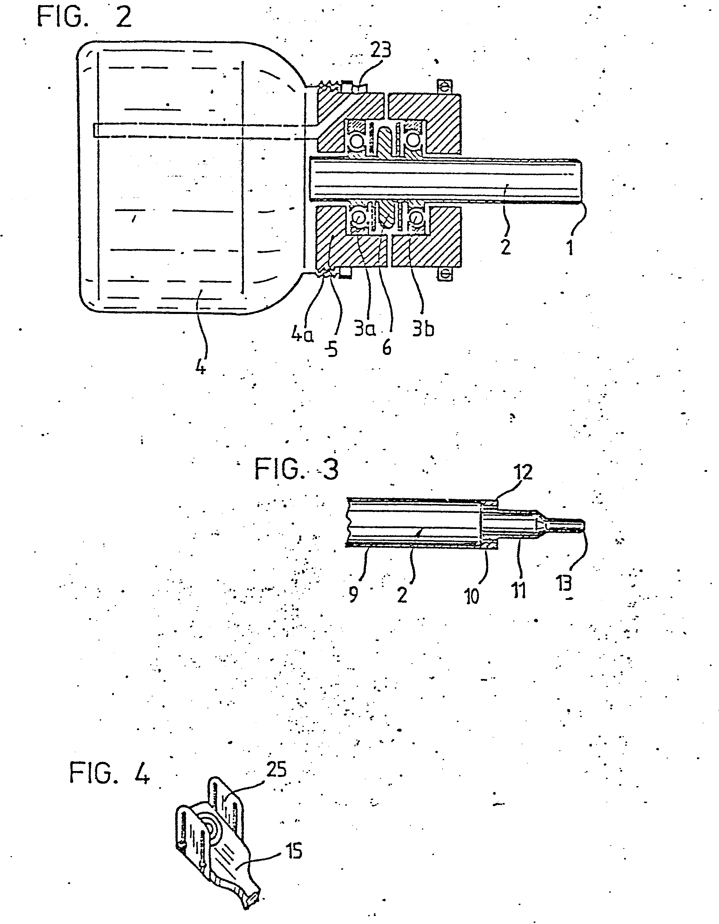

Aus Figur 2 ist eine besondere Ausführungsform der Befestigung des Rundschneidezylinders und des Sammelbehälters 4 sowie eine besondere Ausführungsform einer Drehvorrichtung 6 für den Rundschneidezylinder 2 dargestellt.
Es ist dabei eine Halterung 5 vorgesehen, an welcher einerseits der Sammelbehälter 4 über einen Bajonett- oder
Schraubverschluß 4a befestigt ist. In der Halterung ist
über Kugellager 3a und 3b der Rundschneidezylinder 2
drehbar, jedoch axial unverschieblich gelagert. Zudem ist
in einem Hohlraum der Halterung 5 die Drehvorrichtung 6
angeordnet, die hier aus einer um den Schneidezylinder 2
angeordneten Ankerwicklung 6 besteht. In diesem Fall ist
der Rundschneidezylinder 2 die Ankerwelle der elektrisch
angetriebenen Drehvorrichtung. Des weiteren ist die Anordnung des Rohres 23 der mit Vakuum arbeitenden Auswerfeinrichtung im Sammelbehälter 4 und der Halterung 5
dargestellt.

Figur 3 zeigt einen zweigeteilten Rundschneidezylinder
2, welcher aus einem Drehzylinder 9 besteht, der an
seinem vorderen Ende mit einem Innengewinde 10 ausgestattet ist, in welches ein Reduzierzylinder 11 mit
seinem Gegengewinde 12 eingeschraubt ist. Der Reduzierzylinder weist an seinem anderen, äußeren Ende eine
Schneide 13 auf.

Figur 4 zeigt das äußere Ende des kurzen Schenkel-Teilstückes 15, an welchem kein Amboß vorgesehen ist, sondern direkt in dem angeflachten Ende die Ringvertiefungen zum Eingreifen der Schneiden des Rundschneidezylinders eingebracht sind. An den beiden schmalen Längsseiten des Schenkels 15 sind zwei beispielsweise über Langloch und Schrauben höhenverschieblich verstellbare Wangen 25 angeordnet, die zur genauen Probenentnahme dienen.

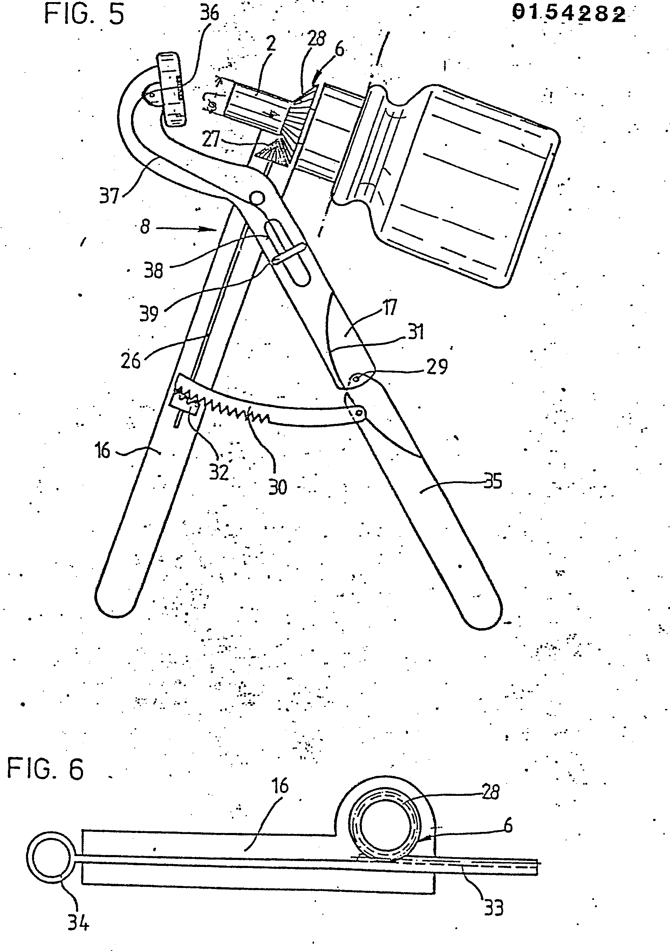

Ein weiteres Ausführungsbeispiel der erfindungsgemäßen
Vorrichtung zeigt Figur 5. Hier ist die Drehvorrichtung
6 eine rein mechanisch arbeitende Vorrichtung, welche
aus Hebel- und Zahnelementen zusammengesetzt ist. Danach ist auf der Mantelfläche des Rundschneidezylinders
ein Zahnkranz 28 angeordnet, welcher mit einem Ritzel
27 kämmt. Das Ritzel 27 ist am äußeren Ende einer Welle
26 befestigt, die ihrerseits drehbar, jedoch unverschieblich an dem den Rundschneidezylinder 2 tragenden Zangenschenkel 16 angeordnet ist. Am unteren Ende der Welle 26 ist ein weiteres Ritzel 23 befestigt, welches seinerseits mit der Verzahnung einer Zahnstange 30 zusammenwirkt. Die Zahnstange 30 ist mit ihrem anderen Ende an einem Schwenkteil 35 angebracht, welches über ein Gelenk 29 mit dem zweiten Zangenschenkel 17 schwenkbar in Richtung auf den ersten Schenkel 16 verbunden ist. Eine relativ starre Blattfeder 31 hält den Schenkel 17 und den Schwenkteil 35 in geradliniger Ausrichtung zueinander.

Als Auswurfvorrichtung ist in diesem Ausführungsbeispiel ein elastisches, flexibles Stahlkabel 37 verdrillten Drähten vorgesehen. An seinem einen Ende weist das Kabel 37 einen Schiebeknopf 39 auf, welcher in einem im Zangenschenkel 17 vorgesehenen Langloch 38 verschieblich angeordnet ist. An seinem anderen Ende weist das Kabel 37 einen zylinderförmigen, in dem Amboß 18 eingelassenen Stempel 36 auf. Nach beendigtem Schneidevorgang gleitet der Stempel 36 durch ruckartiges Vorschieben des Schiebeknopfes 39 so weit in den Rundschneidezylinder 2, daß in dessen lichter Weite befindliches Material in den Sammelbehälter 4 gestoßen wird. In diesem Falle empfiehlt sich die Verwednung eines Rundschneidezylinders 2, dessen lichte Weite sich in Richtung zum Sammelbehälter 4 hin licht konisch weitet.

Figur 6 zeigt eine mechanische Drehvorrichtung 6, welche aus einem am Rundschneidezylinder 2 angebrachten Zahnkranz 28 besteht, der mit der Verzahnung einer Zahnstange 33 zusammenarbeitet. Am anderen, aus dem Schenkel
16 herausragenden Ende der Zahnstange ist eine Fingeröse
34 angeordnet, über welche die Zahnstange 33 in Hin- und
Herbewegung versetzt werden kann, so daß über den Zahnkranz 28 der Rundschneidezylinder 2 in eine Hin- und Her-
drehbewegung versetzt wird.

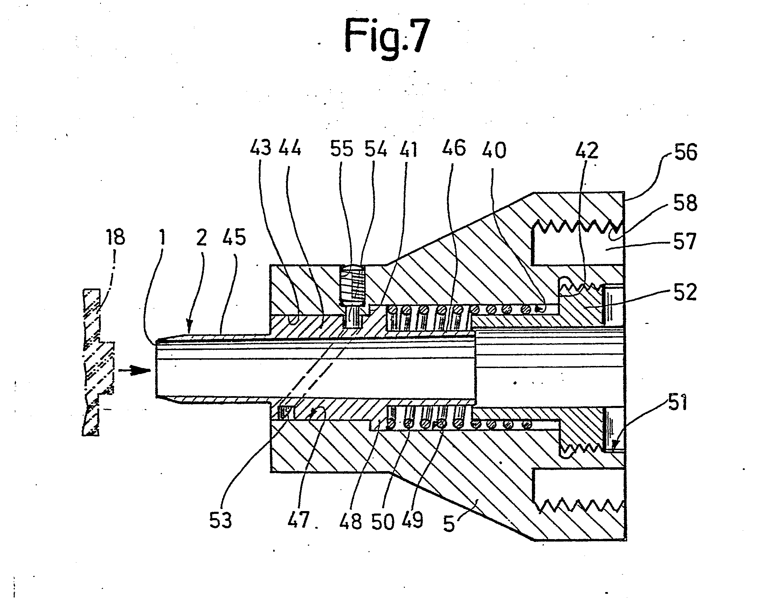

Bei dem in Figur 7 dargestellten Ausführungsbeispiel
sind nur wesentliche Teile gezeigt, nämlich der Rundschneidzylinder 2 mit einem Schneidenrand 1, die diesen
aufnehmende Halterung 5 und teilweise das Gegendruckstück 18. Diese Teile können beispielsweise in der in
Figur 1 beschriebenen Weise an zwei zangenförmig ausgebildeten Griffbranchen gehalten sein.

Die Halterung 5 weist eine sich nach hinten stufenförmig erweiternde Bohrung 40 auf, die eine vordere Stufe
41 und eine hintere Stufe 42 ausbildet. In diese Bohrung 40 ist von der Rückseite her der Rundschneidzylinder 2 eingeschoben, dessen Mittelteil 44 einen größeren
Außendurchmesser aufweist als der Vorderteil 45 und der
rückwärtige Teil 46. Der Mittelteil 44 liegt flächig an
der Innenwand 43 des vorderen Bereiches 47 der Bohrung
40 an, welcher von den verschiedenen Bereichen der Bohrung 40 den geringsten Innendurchmesser aufweist.

Am hinteren Ende des Mittelteils 44 steht ein Kragen 48
radial hervor, der sich im mittleren Bereich 49 der Bohrung 40 befindet. An diesem Kragen 48 stützt sich eine Schraubenfeder 50 ab, die den rückwärtigen Teil 46 des
Rundschneidzylinders 2 umgibt und deren anderes Ende
durch eine in den hinteren Bereich 51 der Bohrung 40
eingeschraubte Schraube 52 im mittleren Bereich 49 gegen axiale Verschiebung fixiert ist.

Im Mantel des Mittelteils 44 des Rundschneidzylinders
2 ist eine wendelförmig umlaufende Nut 53 eingelassen,
in die ein Stift 54 eintaucht, der in eine radiale Gewindebohrung 55 in der Halterung 5 eingeschraubt ist.

Die Bohrung 40 wird auf der hinteren Seite der Halterung von einer zur rückwärtigen Stirnseite 56 offenen
Nut 57 umgeben, deren äußere Seitenwände 58 mit einem
Gewinde versehen sind. In diese Nut kann der in Figur
7 nicht dargestellte Sammelbehälter eingeschraubt werden.

Im Ruhezustand wird der Rundschneidzylinder 2 durch die
Schraubenfeder 50 so weit gegen das Gegendruckstück 18
verschoben, bis der Kragen 48 an der vorderen Stufe 41
der Bohrung 40 anliegt, wie dies in Figur 7 dargestellt
ist. Drückt man den Rundschneidzylinder 2 unter Zwischenlage des zu schneidenden Gutes gegen das Gegendruckstück
18, so wird der Rundschneidzylinder 2 gegen die Kraft
der Schraubenfeder 50 in die Bohrung 40 hineingeschoben,
wobei der Rundschneidzylinder 2 durch den in die wendelförmige Nut 53 eingreifenden Stift 54 gleichzeitig um
seine Längsachse verdreht wird. Man erhält also die Rotationsbewegung des Rundschneidzylinders zwangsläufig dadurch, daß der Rundschneidzylinder 2 beim Annähern der beiden Branchen in axialer Richtung in der Halterung 5 verschoben wird.

Außer zu den eingangs bereits aufgezeigten Verwendungsarten kann die erfindungsgemäße Vorrichtung auch zur Erzeugung von Öffnungen oder Hohlräumen oder zur Gewinnung von Material dienen.

Professor Dr. G. Pfeiffer, 8057 Eching

Vorrichtung zur Entnahme von

Materialproben

Patentansprüche :

1. Vorrichtung zur Entnahme von Materialproben, insbesondere zur Entnahme von tierischen Muskel- oder Gewebeproben, mit einem Rundschneidezylinder (2), der
   an seinem einen Ende eine angeschliffene Schneide (1)
   aufweist,
   d a d u r c h   g e k e n n z e i c h n e t, daß der
   Rundschneidzylinder (2) um seine Längsachse rotierbar
   an einer Haltevorrichtung (8) befestigt ist und daß
   eine den Rundschneidezylinder (2) in Drehbewegung
   versetzende Drehvorrichtung (6) vorgesehen ist.

2. Vorrichtung nach Anspruch 1, dadurch gekennzeichnet, daß zur Aufnahme der ausgeschnittenen Proben ein Sammelbehälter (4) vorgesehen ist, der am rückwärtigen Ende des Rundschneidezylinder (2) lösbar befestigt ist.

3. Vorrichtung nach Anspruch 2, dadurch gekennzeichnet, daß der Sammelbehälter (4) am Rundschneidezylinder (2) über einen Bajonettverschluß (4a) befestigt ist.

4. Vorrichtung nach Anspruch 2, dadurch gekennzeichnet, daß der Sammelbehälter (4) mit dem Rundschneidezylinder (2) über einen Schraubverschluß (4a) verbunden ist.

5. Vorrichtung nach Anspruch 1 und 2, dadurch gekennzeichnet, daß der Rundschneidezylinder (2) in axialer Richtung zweigeteilt ist, wobei der erste, vordere Teil ein Reduzierzylinder (11) ist, welcher der eigentliche Schneidezylinder (2, 1) ist, und der zweite, rückwärtige Teil ein Drehzylinder (9) ist, der das Verbindungsteil mit der Drehvorrichtung (6) und dem Sammelbehälter (4) bildet, und daß die Reduzierzylinder (11) und Drehzylinder (9) lösbar miteinander verbunden sind.

6. Vorrichtung nach Anspruch 1, dadurch gekennzeichnet, daß die Haltevorrichtung (8) ein Griffarm ist, an
welchem der Rundschneidezylinder (2) senkrecht dazu
befestigt ist.


7. Vorrichtung nach Anspruch 1, dadurch gekennzeichnet,
daß die Haltevorrichtung (8) ein Tischstativ ist.


8. Vorrichtung nach Anspruch 1, dadurch gekennzeichnet,
daß die Haltevorrichtung (8) eine zangenförmige Hilfsvorrichtung ist, an deren erstem Schenkelteil (14, 16)
der Rundschneidezylinder (2) drehbar befestigt ist
und an deren zweitem Schenkelteil (15, 17) ein mit
der Stirnseite des Rundschneidezylinders zusammenwirkendes Gegendruckstück (18) angebracht ist.


9. Vorrichtung nach Anspruch 8, dadurch gekennzeichnet,
daß das Gegendruckstück ein Amboß (18) ist, welcher
mindestens eine mit der Schneide (1) des Rundschneidezylinders (2) zusammenwirkende Ringvertiefung aufweist.


10. Vorrichtung nach Anspruch 9, dadurch gekennzeichnet,
daß der Amboß (18) aus einem härteren Metall als der
Rundschneidezylinder gefertigt ist.


11. Vorrichtung nach Anspruch 9, dadurch gekennzeichnet, daß der Amboß (18) aus Kunststoff gefertigt ist und auswechselbar am Schenkelteil (15) befestigt ist.

12. Vorrichtung nach Anspruch 9, dadurch gekennzeichnet, daß beidseits und planparallel am Amboß (18) höhenverstellbare Wangen (25) angeordnet sind.

13. Vorrichtung nach Anspruch 9, dadurch gekennzeichnet, daß im Amboß (18) eine Auswurfeinrichtung vorgesehen ist.

14. Vorrichtung nach Anspruch 13, dadurch gekennzeichnet, daß die Auswurfeinrichtung aus einer zentrischen Öffnung (19) besteht, welche über ein Überdruckventil (22) mit einer Druckluftleitung (21) verbunden ist, und daß der Sammelbehälter (4) druckausgleichende Öffnungen (20) aufweist.

15. Vorrichtung nach Anspruch 1 und 13, dadurch gekennzeichnet, daß die Auswurfeinrichtung aus einem im Sammelbehälter angeordneten gelochten Rohr (23) besteht, welches mit einem Unterdruckrohr (24) in Verbindung steht, und daß die zentrische Öffnung (19) im Amboß (18) als Druckausgleichsöffnung ausgebildet ist.

16. Vorrichtung nach Anspruch 1 und 13, dadurch gekennzeichnet, daß die Auswurfeinrichtung aus einem im
Amboß (18) eingelassenen Stempel (36) besteht, welcher über ein Stahlkabel (36) mit einem in einem
Langloch (38) des Zangenschenkels (17) verschieblich
befestigten Schiebeknopf (39) verbunden ist.

17. Vorrichtung nach Anspruch 1, dadurch gekennzeichnet,
daß die Drehvorrichtung (6) einen elektrischen Antrieb ausweist, wobei eine Ankerwicklung um den Rundschneidezylinder, der gleichzeitig Ankerwelle ist,
angeordnet ist.

18. Vorrichtung nach Anspruch 1, dadurch gekennzeichnet,
daß die Drehvorrichtung (6) einen pneumatischen Antrieb aufweist, wobei an dem Rundschneidezylinder
Turbinenschaufeln angeordnet sind.

19. Vorrichtung nach Anspruch 1, dadurch gekennzeichnet,
daß die Drehvorrichtung (6) eine mechanische Einrichtung ist.

20. Vorrichtung nach Anspruch 19, dadurch gekennzeichnet,
daß die Drehvorrichtung eine zwei-hand-bedienbare
Vorrichtung ist.

21. Vorrichtung nach Anspruch 20, dadurch gekennzeichnet, daß die Drehvorrichtung eine über den Sammelbehälter (4) betätigbare Einrichtung ist.

22. Vorrichtung nach Anspruch 20, dadurch gekennzeichnet, daß die Drehvorrichtung (6) aus einem Drehring besteht, der am Rundschneidezylinder (2) befestigt ist.

23. Vorrichtung nach Anspruch 20, dadurch gekennzeichnet, daß die Drehvorrichtung (6) aus einem am Rundschneidezylinder (2) angeordneten Zahnkranz (28) und einer an einem Schenkel (14, 16) angeordneten Zahnstange (33) besteht, wobei die Zahnstange (33) an ihrem einen Ende eine mit dem Zahnkranz (28) zusammenwirkende Verzahnung aufweist, während an ihrem anderen, aus dem Schenkel (16) herausragenden Ende eine Fingeröse (34) vorgesehen ist.

24. Vorrichtung nach Anspruch 19, dadurch gekennzeichnet, daß die Drehvorrichtung (6) eine ein-hand-betätigbare Vorrichtung ist, die zusammengesetzt ist aus einem am Rundschneidezylinder (2) angeordneten Zahnkranz (28), einer am Schenkel (14, 16) drehbar befestigten Welle (66), welche an ihren beiden Enden je ein Zahnritzel (27, 32) aufweist, wobei das erste Ritzel (27) in den Zahnkranz (28) eingreift, während das zweite Ritzel (32) mit einer am zweiten Schenkel (15, 17) befestigten Zahnstange (30) trieblich verbunden ist.

25. Vorrichtung nach Anspruch 24, dadurch gekennzeichnet, daß das untere, mit der Zahnstange (30) verbundene Ende des zweiten Schenkels (17) ein Schwenkteil (35) ist, das über ein Gelenk (29) mit dem Schenkel (17) in Richtung auf den ersten Schenkel (14, 16) hin schwenkbar befestigt ist.

26. Vorrichtung nach Anspruch 25, dadurch gekennzeichnet, daß am Gelenk (29) eine den Schenkel (17) und das Schenkel-Schwenkteil (25) in unbetätigtem Zustand der Vorrichtung in gradliniger Ausrichtung haltende Blattfeder (31) angeordnet ist.

27. Vorrichtung nach einem der Ansprüche 8 bis 16 und 19, dadurch gekennzeichnet, daß der Rundschneidzylinder (2) axial verschieblich gelagert und durch Federmittel (50) bis zu einem Anschlag (Stufe 41) in Richtung auf das Gegendruckstück (18) verschoben ist und daß eine Zwangsführung (Nut 53, Stift 54) vorgesehen ist, die den Rundschneidzylinder (2) beim Verschieben gegen die Kraft der Federmittel (50) um seine Längsachse in Drehung versetzt.

28. Vorrichtung nach Anspruch 27, dadurch gekennzeichnet, daß die Zwangsführung eine wendelförmige Führung (Nut 53) am Rundschneidzylinder (2) oder an dessen Halterung (5) und einen in diese Führung (Nut 53) eingreifenden Vorsprung (Stift 54) am jeweils anderen Teil
aufweist.


29. Vorrichtung nach Anspruch 27, dadurch gekennzeichnet,
daß Rundschneidzylinder (2) und Halterung (5) ineinandergreifende, wendelförmige Rippen aufweisen.

------------------------

FIG. 1

0154282

FIG. 2

FIG. 3

FIG. 4

FIG. 5

0154282

FIG. 6

# Fig.7